Europäisches Patentamt

European Patent Office

Office européen des brevets

(11) Veröffentlichungsnummer: **0 170 198**
**B1**

(12) **EUROPÄISCHE PATENTSCHRIFT**

(45) Veröffentlichungstag der Patentschrift: **16.05.90**

(51) Int. Cl.⁵: **A 61 M 15/08**

(21) Anmeldenummer: **85109260.1**

(22) Anmeldetag: **24.07.85**

(54) Spender zur intranasalen Applikation von Flüssigkeiten in Form von Sprays oder Tropfen.

(30) Priorität: **01.08.84 DE 8422872 u**

(43) Veröffentlichungstag der Anmeldung:
**05.02.86 Patentblatt 86/06**

(45) Bekanntmachung des Hinweises auf die
Patenterteilung:
**16.05.90 Patentblatt 90/20**

(84) Benannte Vertragsstaaten:
**AT BE CH DE FR GB IT LI LU NL SE**

(56) Entgegenhaltungen:
**DE-A-2 604 001**
**US-A-2 519 555**
**US-A-3 066 669**

(73) Patentinhaber: **Anasco GmbH**
**Aarstrasse 1**
**D-6200 Wiesbaden (DE)**

(72) Erfinder: **Haupt, Joachim, Dipl.-Kfm.**
**Kleine Weinbergstrasse 1**
**D-6200 Wiesbaden (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Bei der intranasalen Applikation von Wirkstofflösungen, welche vorzugsweise im Bereich des Rachens und der Nasenhöhlen wirken, ist es aus medizinischer Sicht wünschenswert, die Behandlung im Liegen durchzuführen. Bedingt durch die horizontale Lage des Patienten gelangt die Wirkstofflösung direkt in den Rachenraum. Durch seitliches Drehen des Kopfes können die Nasennebenhöhlen ebenfalls mit der Wirkstofflösung in Kontakt kommen. Bei der Anwendung im Stehen oder Sitzen, ist es notwendig den Kopf weit nach hinten zu beugen, um einen ähnlichen Effekt zu erreichen.

Meistens werden Wirkstofflösungen, wie etwa Nasentropfen oder Sprays, nicht durch den Arzt sondern von den Patienten selbst angewandt. Um einen den medizinischen Erfordernissen entsprechenden Gebrauch zu gewährleisten, kommt der Gestaltung des Spenders eine besondere Bedeutung zu.

In den letzten Jahren haben sich handbetriebene Zerstäuberpumpen als Spender für die intranasale Applikation von Wirkstofflösungen zunehmend am Markt durchgesetzt. Die Spender bestehen aus einer Vorratsflasche mit Dosierpumpe und einem Nasenadapter. Die Pumpen sind so ausgerichtet, daß nur bei senkrechter Haltung der Flansche der gesamte Füllinhalt abgegeben wird.

Dies und die senkrechte Montage des Nasenadapters verleitet den Anwender den Spender senkrecht oder nur leicht angewinkelt in die Nase einzuführen. Dadurch ist es nicht möglich, derartige Spender zur Applikation von Wirkstofflösungen in Tropfenform zu verwenden, weil die Lösung sofort durch die vordere Nasenhöhle ablaufen würde. Selbst wenn die Lösung nicht als Tropfen sondern als Spray eingesetzt werden soll und der Spender mit einem Sprühventil ausgerüstet ist, läuft der sich in der vorderen Nasenhöhle niederschlagende Sprühnebel wirkungslos ab.

Aus der DE—A—2 604 001 ist ein Spender zur intranasalen Applikation von Wirkstofflösungen bekannt. Er besteht aus einer Vorratsflasche mit Dosierpumpe und aufgesetztem Nasenadapter. Der Gegenstand der vorliegenden Anmeldung unterscheidet sich von dem Spender der DE—A—2 604 001 dadurch, daß der Nasenadapter in einem Winkel von 20 bis 70° gegen die Mittelachse des Spenders geneigt ist und daß der Nasenadapter an seiner oberen Seite eine Grifffläche aufweist. Durch seine Gestaltung löst der neue Spender die Aufgabe, eine Vorrichtung zur Applikation von Flüssigkeiten in Form von Sprays oder Tropfen durch die Nase zu entwickeln, der so angeformt ist, daß bei seiner Anwendung der Benutzer die aus medizinischer Sicht günstige Kopfhaltung einnimmt. Der oben erwähnte bekannte Spender kann dagegen nicht in dieser Kopfhaltung benutzt werden.

Die nasale Applikation ist bei vielen Wirkstoffen angebracht, insbesondere dann, wenn diese Wirkstoffe a) lokale Wirkung im HNO-Bereich entfalten oder b) die Wirkstoffe bei der Passage durch den Magen-Darm-Trakt in ihrer Wirkung beeinträchtigt werden.

Zur Gruppe a) gehören zahlreiche schleimhautabschwellende Mittel, wie Xylometazolin-Hydrochlorid, Tramazolin-Hydrochlorid, Indanazolin Hydrochlorid, Oxymetazolin-Hydrochlorid, Naphazolinnitrat, Tetryzolin-Hydrochlorid, Fenoxyzolin-Hydrochlorid, oder Anti-Schnarchmittel wie sie etwa in den Deutschen Patentanmeldungen DE—33 17 558, DE—33 17 530, DE—31 46 570, DE—31 52 962, DE—30 46 484 oder der französischen Anmeldung FR—83 14 445 sowie in den europäischen Anmeldungen EP—109 857, EP—105 287, EP—110 550 und EP—137 302 beschrieben sind. Anti-Schnarchmittel sollen einer Austrocknung der rhino-pharyngitischen Schleimhäute entgegenwirken.

Wirkstoffe der Gruppe b) die oral verabreicht bei der Passage durch den Magen-Darm-Trakt in ihrer Wirkung beeinträchtigt werden, sind insbesondere Peptide wie etwa Interferon oder Bruserelin für die Applikationen über die Schleimhäute vorgeschlagen werden.

Sowohl für Wirkstoffe der Gruppe a) als auch für Wirkstoffe der Gruppe b) hat die Applikation als Spray den Nachteil, daß ein Teil des Sprühnebels durch die Atmung in die Lage gelangt. Für eine gezielte nasale Applikation ist daher die Verabreichung von Tropfen bevorzugt.

Der Erfindung liegt somit die Aufgabe zugrunde einen Spender zur Applikation von Flüssigkeiten in Form von Sprays oder Tropfen durch die Nase zu entwickeln, der so angeformt ist, daß bei dessen Anwendung der Benutzer die aus medizinischer Sicht günstigste Kopfhaltung einnimmt und die es zugleich ermöglicht die Wirkstofflösung sowohl als Tropfen oder gewünschtenfalls auch als Spray zu verabreichen.

Die Aufgabe wird erfindungsgemäß gelöst durch einen handbetriebenen Spender in Überkopfausführung zu intranasalen Applikation von Wirkstofflösungen, bestehend aus einer Vorratsflasche (1) mit Dosierpumpe (2) und einem auf diese Dosierpumpe (2) aufgesetzten Nasenadapter (3), dadurch gekennzeichnet, daß der Nasenadapter (3) in einem Winkel von 20° bis 70° gegen die Mittelachse (12) des Spenders geneigt ist und daß der Nasenadapter (3) an seiner oberen Seite eine Grifffläche (11) aufweist und unter Druck auf diese Fläche (11) um die Strecke (h) längs der Mittelachse (12) verschiebbar ist und auf die Pumpe (2) wirkt.

Die Wirkungsweise des Spenders wird im folgenden anhand der beigefügten Zeichnungen erläutert, wobei die Zeichnungen, ohne den Umfang des Anmeldegegenstandes zu beschränken, eine bevorzugte Ausführungsform des erfindungsgemäßen Spenders zeigen:

Figur 1: Einen Spender mit Verschlußkappe im fertig montierten Zustand

Figur 2: Eine Projektion des Umrisses der einzelnen Teile des Spenders längs der Mittelachse

Figur 3: Ein Spender in betriebsbereiter Überkopfstellung

Figur 4: Den oberen Teil des Spenders mit Überkopf-Dosierpumpe (2) und Nasenadapter

Figur 5: Darstellungen der Kopf- und Handhaltung bei Anwendung des Spenders

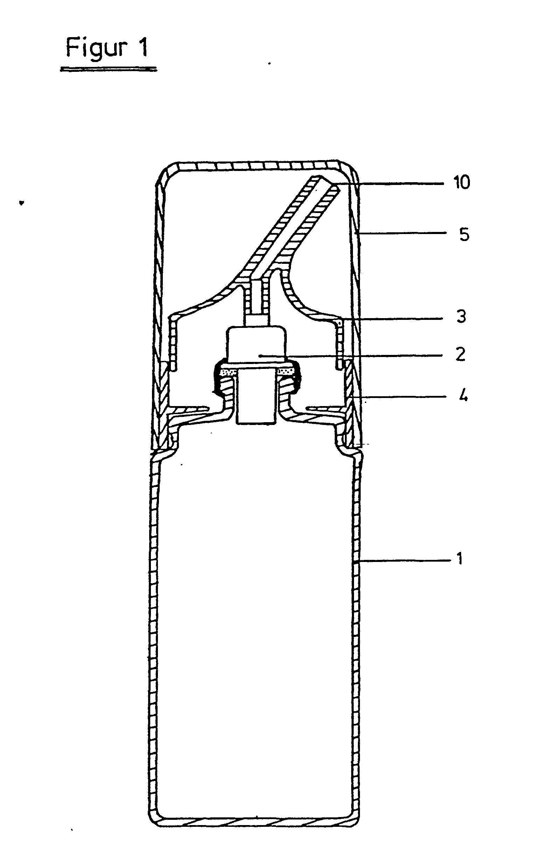

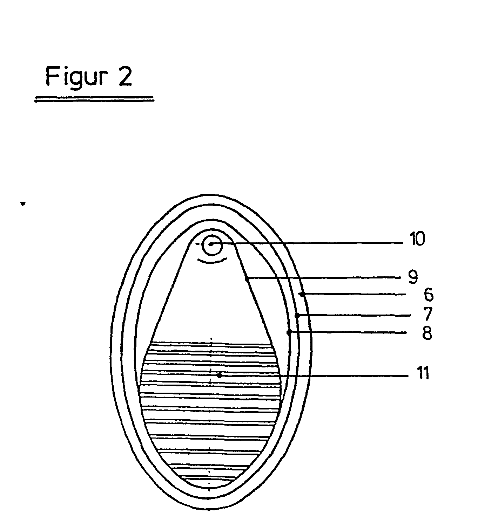

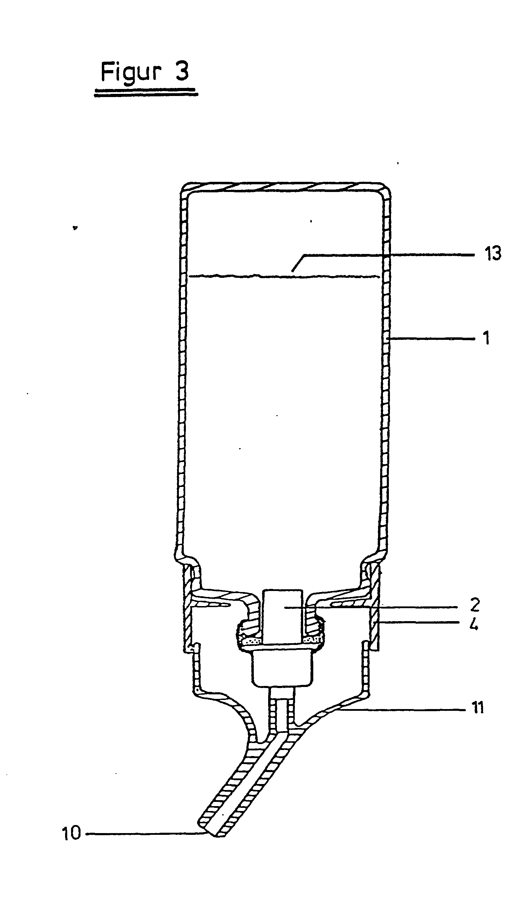

Der in Figur 1 und 3 dargestellte erfindungsgemäße Spender besteht aus einer Flasche (1) mit einer in Überkopflage arbeitenden Dosierpumpe (2) und einem diese Dosierpumpe betätigenden Nasenadapter (3), sowie einen auf die Flasche (1) aufgesetzten Zwischenstück (4), welches die bündig mit der Außenseite der Flasche (1) abschließende Schutzkappe (5) trägt. Die äußere Form des Spenders kann von beliebigen Grundformen hergeleitet werden, hervorzuheben sind kreisförmige, rautenförmige oder trigonale Grundmuster, bevorzugt ist die elipsoide Grundform, wie sie Figur 2 in Projektion längs der Mittelachse zeigt.

Es bedeuten Linie (6) den äußeren Umfang der Flasche (1) und der Schutzklappe (5) und Linie (7) den äußeren Umfang des Zwischenstücks (4), Linie (8) kennzeichnet den Umfang des unteren Teils des Nasenadapters (3) dessen oberer Teil sich längs der Linie (9) zur Mündung (10) hin verjüngt, die Schraffierung in dem von der Linie (9) umschlossenen Teil gibt die Lager der Griffläche (11) an.

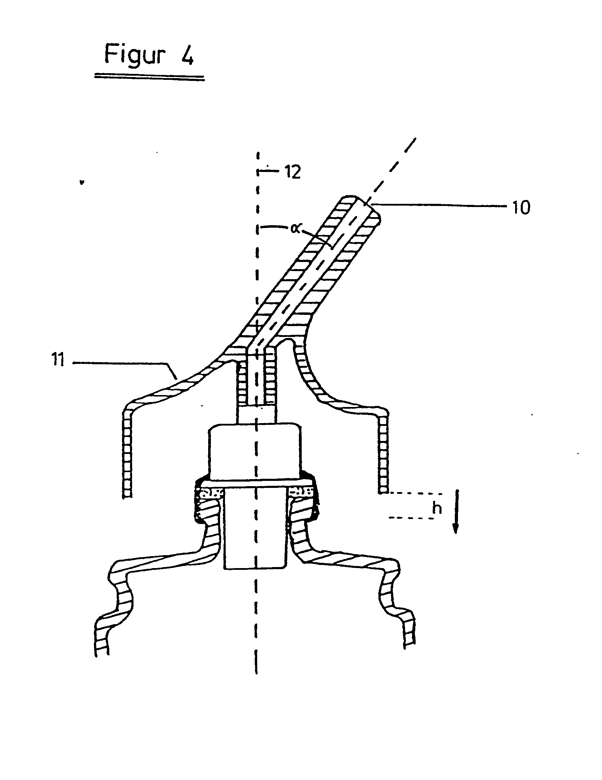

Der obere Teil des Nasenadapters (3) ist, wie in Figur 4 gezeigt, um einen Winkel (α) gegen die Mittelachse (12) des Spenders geneigt. Der Neigungswinkel kann im Bereich von 20° bis 70°, vorzugsweise jedoch zwischen 30° und 45°, gewählt werden. Durch Druck auf die Griffläche (11) kann der Nasenadapter (3) um die Strecke (h) nach unten verschoben werden und wirkt so auf die Dosierpumpe, daß eine dem Hub (h) entsprechende Menge des Füllguts (13) gefördert wird.

Die Beschreibung der Handhabung des erfindungsgemäßen Spenders zeigt, daß durch das Zusammenwirken der Einzelelemente die erfindungsgemäße Aufgabe gelöst wird:

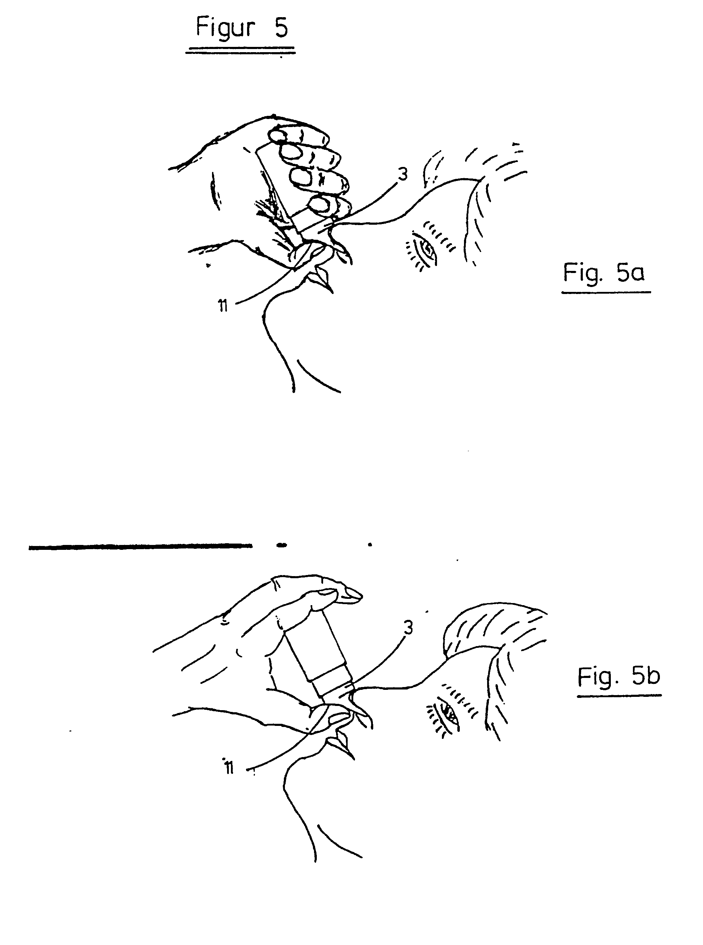

Zur Benutzung des Spenders wird wie in Figur 5a und 5b gezeigt, der Nasenadapter in die vordere Nasenhöhle eingeführt. Weil die Dosierpumpe in Überkopflage das Füllgut (13) fördert, ist der Anwender gezwungen den Kopf nach hinten zu neigen oder die Anwendung im Liegen durchzuführen. Diese aus medizinischer Sicht wünschenswerte Haltung bei der Applikation wird auch dadurch unterstützt, daß der Spender am einfachsten dann betätigt werden kann, wenn der Daumen auf der oberen Griffläche (11) des Nasenadapters (3) liegt und mit den restlichen Fingern der Hand die Flasche (1) gehalten wird (Fig. 5a). Besonders günstig kann der erfindungsgemäße Spender dann gehalten werden, wenn Zeige- und/oder Mittelfinger auf dem Flaschenboden und der Daumen auf der Griffläche (11) des Nasenadapters (3) liegen (Fig. 5b).

So können auch Personen, welche in ihrer Beweglichkeit stark eingeschränkt sind den Spender sicher handhaben.

Dadurch, daß die Griffläche (11) im Bereich der Mittelachse (12) im Winkel (α) (oder nahezu im Winkel α) gegen die Mittelachse geneigt ist, kann der erfindungsgemäße Spender nur dann gut bedient werden, wenn der Anwender mit dem Daumen auf die Griffläche drückt (Fig. 5). Nur mit dem Daumen als stärksten Finger der Hand ist es möglich eine geneigte Fläche um die für den Hub h erforderliche Strecke längs der Mittelachse (12) zu verschieben, wobei die anderen Finger der Hand, wie in Figur 5a und 5b gezeigt den Spender festhalten.

Wenn der Daumen auf der Griffläche (11) aufliegt, kann der erfindungsgemäße Spender nur dann gehandhabt werden, wenn der Kopf zurückgelegt wird. In allen anderen Stellungen ist ein Einbringen der Spendeöffnung (10) in die Nase nicht möglich.

Weil durch die besondere Formgebung und Funktionsweise des erfindungsgemäßen Spenders die intranasale Applikation von Wirkstofflösungen entweder im Liegen oder mit stark nach hinten geneigtem Kopf erfolgt, sind diese Flüssigkeiten nicht nur als Sprays, sondern auch in Tropfenform applizierbar. Die Abgabe von Tropfen durch die vordere Nasenhöhle in den Rachenraum war bisher nur bei Verwendung von Pipetten möglich. Nachteile der intranasalen Applikation von Flüssigkeiten durch Pipetten sind: mangelnde Dosiergenauigkeit, unsichere Handhabe durch den Patienten und mangelnde Hygiene bei mehrmaliger Anwendung der Pipette.

Demgegenüber ist es mit dem erfindungsgemäßen Spender erstmals möglich Wirkstofflösungen intranasal in Tropfenform, bei höchster Gebrauchssicherheit durch den Patienten, genau dosiert zu verabreichen, wobei die Flüssigkeit in der Flasche unter hygienisch einwandfreien Bedingungen eingeschlossen ist. Der erfindungsgemäße Spender kann aber auch zur intranasalen Applikation von Wirkstoffen in Form von Sprays oder Aerosolen Verwendung finden. Durch die Wahl geeigneter Pumpen (bspw. Dosier- und Zerstäuberpumpen) wird bestimmt, in welcher Weise das Fördergut durch den Spender in die Nasenhöhle abgegeben wird.

1 Vorratsflasche
2 Dosierpumpe
3 Nasenadapter
4 Zwischenstück
5 Schutzkappe
6 äußerer Umfang der Flasche (1)
7 äußerer Umfang Zwischenstück (4)
8 Umfang des unteren Teils des Nasenadapters (3)
9 Zur Mündung (10) hin verjüngender Teil des Nasenadapters
10 Mündung des Nasenadapters
11 Griffläche
12 Mittelachse
13 Füllgut

## Patentansprüche

1. Handbetriebener Spender in Überkopfausführung zur intranasalen Applikation von Wirkstofflösungen, bestehend aus einer Vorratsflasche (1) mit Dosierpumpe (2) und einem auf diese Dosierpumpe (2) aufgesetzten Nasenadapter (3), dadurch gekennzeichnet, daß der Nasenadapter (3) in einem Winkel (α) von 20° bis 70° gegen die Mittelasche (12) des Spenders geneigt ist und daß der Nasenadapter (3) an seiner oberen Seite eine Griffläche (11) aufweist und unter Druck auf diese Fläche (11) um die Strecke (h) längs der Mittelachse (12) verschiebbar ist und auf die Pumpe (2) wirkt.

2. Spender nach Anspruch 1, dadurch gekennzeichnet, daß der Nasenadapter (3) in einem Winkel (α) von 30° bis 45° gegen die Mittelachse (12) des Spenders geneigt ist.

3. Spender nach Anspruch 1 oder 2, dadurch gekennzeichnet, daß die Grifffläche (11) im Bereich der Mittelachse (12) um den Winkel (α) gegen die Mittelachse (12) geneigt ist.

## Revendications

1. Distributeur utilisable manuellement au-dessus de la tête pour l'administration intranasale de solutions de substances actives, constitué d'un flacon réservoir (1) avec une pompe de dosage (2) et un adaptateur nasal (3) disposé sur cette pompe de dosage (2), caractérisé en ce que l'adaptateur nasal (3) est incliné d'un angle (α) de 20° à 70° par rapport à l'axe (12) du distributeur et en ce que l'adaptateur nasal (3) présente sur son côté supérieur une face de manipulation (11) et par pression sur cette face (11) est déplaçable de l'intervalle (h) selon l'axe (12) et agit sur la pompe (2).

2. Distributeur selon la revendication 1, caractérisé en ce que l'adaptateur nasal (3) est incliné d'un angle (α) de 30° à 45° par rapport à l'axe (12) du distributeur.

3. Distributeur selon la revendication 1 ou 2, caractérisé en ce que le face de manipulation (11) est inclinée dans le domaine de l'axe (12) de l'angle (α) par rapport à l'axe (12).

## Claims

1. Hand operated overhead-operation dispenser for the intranasal administration of solutions of active substance, consisting of a storage flask (1) with a metering pump (2) and a nasal adapter (3) fitted over this metering pump (2), characterised in that the nasal adapter (3) is inclined at an angle (α) of 20° to 70° relative to the central axis (12) of the dispenser and the nasal adapter (3) has a gripping surface (11) on its upper side and when pressure is applied to this surface the adapter is movable by a distance (h) along the central axis (12) and acts on the pump (2).

2. Dispenser according to claim 1, characterised in that the nasal adapter (3) is inclined at an angle (α) of 30° to 45° relative to the central axis (12) of the dispenser.

3. Dispenser according to claim 1 or 2, characterised in that, in the region of the central axis (12), the gripping surface is inclined by the angle (α) relative to the central axis (12).

## Figur 1

10

5

3

2

4

1

Figur 2

# Figur 3

13

1

2
4

11

10

Figur  4

# Figur 5

Fig. 5a

Fig. 5b